# EUROPEAN PATENT APPLICATION

(11) **EP 4 602 972 A1**
(43) Date of publication of application: **20.08.2025**
(21) Application number: 25157377.0
(22) Date of filing: 12.02.2025
(51) Int. Cl.: A43B 17/00, A43D 1/02, A61B 5/103, B33Y 80/00

(54) **FULLY AUTOMATIC AI-BASED MANUFACTURING OF AN INSOLE**

(30) Priority: 15.02.2024 EP 24157782; 22.02.2024 BE 202405106
(71) Applicant: Orthopedie Kestelyn, 9700 Oudenaarde (BE)
(72) Inventor: Dhondt, Kristof, 9700 Oudenaarde (BE)
(74) Representative: Rogiest, Wouter

(57) **Abstract**

The present invention provides, inter alia, a method for designing an insole, comprising receiving a measurement of a foot sole associated with pressure, said measurement preferably being obtained by placing a load on a measurement surface; receiving a sole profile; determining, based on said measurement, one or more anatomical zones; determining, based on said anatomical zones, a foot profile comprising a major axis; determining, at least based on said foot profile and said anatomical zones, an interpolating surface; aligning the interpolating surface with the sole profile, to obtain an aligned 3D profile; generating, based on said aligned 3D profile, an instruction for manufacturing the insole; wherein said determining of the interpolating surface is realized according to a 3D characterization according to respective positions (y1, y2) on said major axis; wherein said determining of the interpolating surface comprises calculating, based on said foot profile and said anatomical zones, a plurality of at least two anchor coordinates (d0-d3) each lying in a respective cross-section corresponding to said respective positions (y1, y2), and calculating, based on said at least two anchor coordinates (d0-d3), an interpolating surface.

## Description

### TECHNICAL FIELD

The invention relates to the design of an insole by means of software, and the related manufacturing of an insole.

### STATE OF THE ART

One problem with the known methods of designing and manufacturing insoles is that they are difficult to automate. This is particularly the case with custom insoles, which are adapted to the user's anatomy. If the insole consists of several parts, automation is also (or additionally) difficult.

A currently common way to design such insoles is by manual annotation, by an expert, of a 2D representation of a pressure measurement. This involves, for example, indicating one or more smooth curves over this pressure measurement, forming a characterization of how the insole should be designed. This can be either a physical printout of this pressure measurement (where colors and/or grey scales indicate the intensity of pressure), or a digital representation on a computer screen (analogous to a printed version and/or via an interactive 3D model, e.g., a CAD model). This characterization is then used as an input for the (either manual or automatic) production of the sole. The production may thereby involve, for example, milling from sandwich blocks, and/or 3D printing of a sole, in each case in accordance with the 3D profile as characterized.

A disadvantage of the annotation of the pressure measurement with curves is that it gives at most an indirect estimate of what the insole should finally look like. Another disadvantage is that these curves are difficult to interpret by non-experts. Another disadvantage is that, in case of changed circumstances, such as a new diagnosis, the expert must reapply these curves himself. Hence, there is a need for an insole design methodology solving these problems and providing for more automation.

Techniques with types of automation are described in (Davia-Aracil et al, 3D printing of functional anatomical insoles, Computers in Industry 95(2): 38-53, 2018, https://doi.org/10.1016/j.compind.2017.12.001), (Davia-Aracil et al, A new methodological approach for shoe sole design and validation; Int J Adv Manuf Technol 86, 3495-3516, 2016, https://doi.org/10.1007/s00170-016-8427-5) and (Marrone et al, An automated system for the design of orthopedic insoles, 2023 IEEE International Symposium on Medical Measurements and Applications (MeMeA), Jeju, Korea, Republic of, 2023, https://doi.org/10.1109/MeMeA57477.2023.10171871). However, the techniques described herein start from a "black box" application of computer techniques, without any insight for the operator. Also, due to the "brute force" application of computing power, these techniques involve long processing times, making design adjustments difficult.

There is a need for methods, devices and systems that allow for a smooth and fast design, which are preferably also user-friendly to the operator. The invention thereby seeks to solve at least one of the above-mentioned problems.

### SUMMARY OF THE INVENTION

The invention aims to solve the above-mentioned problems, by providing a new 3D characterization for insole design, using an interpolating surface, an aligned 3D profile and anchor coordinates. This characterization can inherently be automated and therefore enables greater automation of the process from pressure measurement and diagnosis to the production of the insole.

Specifically, the invention relates to a method, a device, and a system for manufacturing an insole, or, equivalently, designing an insole, wherein the manufacturing is automated as much as possible. In examples, the manufacturing is fully automated, with the input being the measurement and the output being the insole thus designed. Thereby, the invention is advantageously appropriate for incorporating computer vision algorithms and other related AI algorithms and machine learning algorithms into manufacturing. The specific 3D characterization allows efficient calculation of the design, making the design fast, and requiring less computing power than is the case with the "brute force" use of AI algorithms. Thus, the invention provides an AI-friendly method, and, in embodiments, a method being at least partially AI-based.

In a first aspect, the invention provides a method for designing an insole, comprising receipt of a measurement of a foot sole associated with pressure;
receipt of a sole profile;
determination, based on said measurement, of one or more anatomical zones;
determination, based on said anatomical zones, of a foot profile comprising a major axis;
determination, at least based on said foot profile and said anatomical zones, of an interpolating surface;
alignment of the interpolating surface with the sole profile for obtaining an aligned 3D profile;
generation, based on said aligned 3D profile, of an instruction for manufacturing the insole;
wherein said determination of the interpolating surface is done according to a 3D characterization according to respective positions (y1, y2) on said major axis;
wherein said determination of the interpolating surface comprises:
   calculation, based on said foot profile and said anatomical zones, of a plurality of at least two anchor coordinates (d0-d3) each lying in a respective cross-section corresponding to said respective positions (y1, y2), and
   calculation, based on said at least two anchor coordinates (d0-d3), of an interpolating surface.

In embodiments, said measurement, which relates to one or more digital data files, is obtained by placing a load on a digital measurement surface, such as, for example, an electronic pressure mat or an optical-scanning based pressure gauge, which writes the measurement to a digital medium, after which the measurement can be read in for design purposes. In exemplary embodiments, the measurement thereby directly relates to a quantitative pressure measurement, for example in arrangements in which the pressure mat includes electronic sensors which measure the pressure.

In embodiments, said measurement is obtained by placing a load on a non-digital measurement surface, such as, for example, a surface with inks, which may lead to a non-digital measurement, which can then be read in after digitization, e.g., optical scanning, for design purposes. In exemplary embodiments, the measurement thereby directly relates to a quantitative pressure measurement, for example in arrangements in which the local intensity of ink printing is a measure of the local pressure exerted when making the print.

In embodiments, said measurement is obtained by LIDAR measurement, wherein the pressure measurement is measured, for example, indirectly based on LIDAR imaging of the foot sole in an unloaded state (when not leaned on), wherein features of the foot sole surface, such as, for example, the shape of the foot sole and locations and intensity of callus formation, may be indicative of pressure distribution at load, and thus, indirectly, establish a digital and quantitative pressure measurement, which can be read in for design purposes.

In embodiments, in addition to quantitative information related to pressure when measuring the foot sole, the measurement includes quantitative and/or qualitative information related to other aspects, such as a diagnosis and/or a use requirement and/or a use purpose, and so on.

Such a method allows advantageous bridging between pressure measurements of patients, on the one hand, and a characterization of the desired 3D profile of the insole, on the other hand.

The method thereby advantageously allows an operator to understand the design and additionally adjust it. This is facilitated by the advantageous 3D characterization, with a parametric model, and the associated interpolating surface.

The method also allows advantageous modularity. In embodiments, 3D elements are added, with these 3D elements modulating the interpolating surface. This is particularly advantageous in cases where the 3D element is related to a compression-free pixel-based representation, or, equivalently, a lossless bitmap representation. In such cases, both the 3D element and the interpolating surface are readily visualizable and can help the operator when designing the insole. Also, the modulation induced by the 3D element can thereby be understood intuitively, because of the intuitive visualization. Such modularity can be particularly advantageous when the measurement involves a combination of measurement of pressure and further aspects, such as a diagnosis. For example, a patient (i.e., a user) who has already been diagnosed with "hallux rigidus" may require the insole to be adapted to the combination of both pressure measurements and the diagnosis, which is advantageously made possible through modulation of the interpolating surface and/or the aligned 3D profile. In other examples, there may be more than one diagnosis to consider, which is modularly possible through the addition of more 3D elements. In this way, the invention provides a possibility to create a "library" of 3D elements, which can be individually managed and also individually improved, for example based on user feedback.

Another possible advantage is that such a parametric model, with anchor coordinates, corresponds well to the technical characteristics of the desired end product. After all, for an insole, for ergonomic reasons, a uniformly smooth surface is desired, making a characterization using a smooth interpolation advantageous.

A further advantage of the invention may be related to a good 3D characterization of pressure measurement. Unlike characterization based on an equidistant 2D grid or 3D grid, there are (much) fewer values to fit, thus avoiding the typical problems of overfitting, which may advantageously be combined, in embodiments, with AI-based preprocessing of the pressure measurement.

A further possible advantage is related to avoiding problems with quantization. Indeed, the actual 3D profile, with full (x,y,z) positions, can be recalculated on demand each time a different production medium (milling, 3D printing,...) presents itself. Such an advantage is not offered by prior art methods. For example, the interpolating surface can be chosen such that the corresponding point cloud is only quantized at the level of points d0-d3 (e.g., for interpolation where d0-d3 lie on the surface), or even without any quantization (e.g., when the interpolation is done without requiring d0-d3 to lie on the surface).

Another possible advantage is the ease with which an operator can adjust the algorithmic conversion of the pressure measurement and diagnosis to the interpolating surface. Indeed, it can be determined experimentally that a particular diagnosis is not optimally converted to a suitable upper surface of the insole, whereby specifically for that diagnosis, the algorithm can be adjusted. Thereby, all adjustments can be expressed as a function of a limited set of parameters, e.g. just d0-d3, rather than via a complex representation based on a 2D grid or 3D grid.

In a second aspect, the invention relates to a device comprising a processor configured to perform a method according to the invention.

In a third aspect, the invention provides a system comprising:
- the device according to the invention, for generating an instruction for manufacturing the insole; and
- means for receiving said instruction and manufacturing the insole, by milling and/or 3D printing and based on said instruction.

In a fourth aspect, the invention relates to a computer program product comprising memory comprising instructions which, when executed on a processor, cause the processor to implement a method according to the invention.

The second, third and fourth aspects may provide advantages similar to these of the method according to the invention. Embodiments according to the dependent claims and their respective potential advantages are explained in the detailed description.

### DESCRIPTION OF THE FIGURES

**Fig. 1** shows an exemplary embodiment of an insole designed with a method according to the invention. Thereby, Fig. 1A shows a front view in perspective, and Fig. 1B shows a side view in perspective.
**Fig. 2** shows an exemplary embodiment of an interpolating surface obtained when designing an insole according to the invention. Thereby, Fig. 2A shows an example of a cross-section transverse to the major axis, while Fig. 2B shows an example of the interpolating surface in perspective.
**Fig. 3** illustrates an exemplary embodiment in which the foot profile and various anatomical features are determined based on a measurement.
**Fig. 4** shows an example of a 3D element for designing a projecting member.
**Fig. 5** shows an exemplary embodiment of an aligned 3D profile obtained when designing an insole according to the invention, according to various views in perspective. Thereby, Fig. 5A shows a lateral front view, Fig. 5B shows a side view, and Fig. 5C shows a top view.

### DETAILED DESCRIPTION

Unless otherwise defined, all terms used in the description of the invention, including technical and scientific terms, have the meaning commonly understood by those skilled in the technical field of the invention. For a better assessment of the description of the invention, the following terms are explicitly explained.

"A," "the," and "the" refer in this document to both the singular and plural unless the context clearly assumes otherwise. For example, "a segment" means one or more than one segment. The recitation of numeric intervals by endpoints includes all integers, fractions and/or real numbers between endpoints, these endpoints included.

In this document, the term "operator" refers to anyone who interacts with the facilities (computer) to generate an instruction for manufacturing the insole. By extension, the operator may also supervise the pressure measurement and/or the actual manufacturing, which is related, for example, to milling or 3D printing. The operator is thereby supervising, without necessarily requiring any action, since the method is fully automated in embodiments.

In this document, the term "user" refers to the person for whom the insole is intended and with whom the insole and pressure are associated. In embodiments, the user refers to a patient with whom a diagnosis is associated.

In this document, the term "production medium-dependent" should not be construed restrictively, but merely as "appropriate for certain media for the physical manufacturing of the insole," such as "appropriate for 3D printing" and/or "appropriate for milling". This term distinguishes representations, such as 2D grids of height values, or voxel representations, from representations that are (completely) independent of any production medium, such as mathematically ("vectorially") defined surfaces, e.g., the interpolating surface. The term "voxel representation" is to be understood as a generic term for any grid-based representation related to three dimensions. Thus, a 2D grid with grey scales corresponding to various heights is also considered a voxel representation, as is a 3D grid with values associated with each box in space.

In this document, the terms "anchor point" and "anchor coordinates" are interchangeable, i.e., the anchor point refers to nothing but a position relative to an axis system, where the position is fully defined by just its coordinates relative to that axis system.

In embodiments, said determination of the foot profile comprises the determination, based on said anatomical zones, of said major axis, for further determination of said foot profile.

In embodiments, said 3D characterization relates to rectangular cross-sections extending in an x-direction between the same pair of ordinates (x0, x3); and wherein said 3D characterization is based on at least four coordinate-related values comprising a pair of ordinates (x1, x2) and a pair of abscissae (z0, z3) belonging to anchor coordinates (d0-d3).

In embodiments, said determination of the anatomical zones based on the measurement is related to a computer vision algorithm, preferably to a computer vision algorithm based on neural networks.

In embodiments, said determination of the anatomical zones comprises the determination, by means of the computer vision algorithm, of at least one metatarsophalangeal joint, MTP joint. The inventor has found that such anatomical feature, and in particular each of the five MTP joints, is an important reference in determining the general anatomy of the foot. Thereby, the determination of the first MTP joint, and by extension the two extreme MTP joints (first and fifth) proves to be particularly useful in accurately determining the interpolating surface. In embodiments, the zones of the other MTP joints, being the second, third and fourth zone, are not detected based on the measurement itself, but via approximate geometric progression between the five MTP joints, for example with multiplier 2, in good accordance with human anatomy, see for example Fig. 2.8 in (Leemrijse, Besse, Devos Bevernage and Valtin, Pathologie du pied et de la cheville, 2nd edition, Elsevier Masson, 2015).

In embodiments, said determination of the interpolating surface is at least partly based on B-splines, preferably nurbs.

In embodiments, said incompletely aligned 3D profile relates to extending the interpolating surface between predetermined fixed boundaries (x0, x3), and wherein said aligning relates to cutting out the sole profile from the interpolating surface.

In embodiments, said plurality of at least two anchor coordinates (d0-d3) per position each time comprises no more than ten anchor coordinates per position, preferably no more than five.

In embodiments, said plurality of at least two anchor coordinates (d0-d3) per position each relates to, across all positions, no more than 1,000 positions, preferably no more than 500 positions. This can have the advantage of requiring little computing power, with an elegant and lightweight representation, which in turn can lead to faster design and production of insoles. Such a lightweight representation is compact and can avoid overfitting when matching measurement and model. In addition, this representation is conveniently clear, and therefore easily manipulable by an operator.

In embodiments, said determination of the interpolating surface comprises the sub-steps:
calculation of a point cloud;
application, on the point cloud, of a surface reconstruction algorithm, for obtaining the interpolating surface;
wherein said surface reconstruction algorithm is related to triangulation.

In embodiments, said method comprises, at the step of determining the interpolating surface, and/or at the step of generating the instruction, the sub-step:
transcoding, from said interpolating surface and/or said aligned 3D profile, to a production medium-dependent voxel representation;
wherein said generation of the instruction is realized based on the production medium-dependent voxel representation.

In embodiments, said method comprises, at the step of determining the interpolating surface, and/or at the step of generating the instruction, the sub-steps:
receipt of a 3D element characterized by one or more element parameters;
modulation, based on said 3D element, of said interpolating surface and/or said aligned 3D profile, for obtaining a modulated representation;
wherein said generation of the instruction is realized based on the modulated representation.

In embodiments, it is the step of generating the instruction that comprises the sub-steps of
receipt of a 3D element characterized by one or more element parameters; and
modulation, based on said 3D element, of said aligned 3D profile for obtaining a modulated representation;
wherein said generation of the instruction is realized based on the modulated representation;
wherein the 3D element is represented by a lossless bitmap representation; and
wherein the modulation is related to the convolution of the lossless bitmap representation with a production medium-dependent voxel representation of the interpolating surface or the aligned 3D profile.

In embodiments, receiving the sole profile is related to the following sub-steps:
receiving, preferably making, imaging and/or video imaging and/or LIDAR imaging of a shoe (either left shoe, right shoe, or both) for which the insole(s) is (are) intended;
automatically recognizing the make and model and shoe size of shoes, through a computer vision algorithm which correlates the imaging with information contained in a first database;
receiving, based on the recognized make, model and recognized shoe size, of the corresponding sole profile from a search in a second database (which may or may not be different from the first database).

In embodiments, receiving the sole profile is related to the following sub-steps:
receiving, preferably textually but alternatively via imaging comprising the same information textually and/or as a logo, of a shoe size, make and model of a shoe (either left shoe, right shoe, or both) for which the insole(s) is (are) intended;
receiving, based on the make, model and shoe size, of the corresponding sole profile from a search in a second database (which may or may not be different from the first database).

In embodiments, the sole profile comprises at least a maximum outer contour within which the insole should fit to fit a shoe or footwear belonging to the sole profile. In embodiments, the sole profile includes information about a required surface finishing of the bottom of the insole, which may relate to the bottom of the shoe or a surface of the footwear, and/or an indication of whether a flat bottom is sufficient for the insole. In embodiments, the sole profile includes information about the free height within the shoe or footwear.

In embodiments, the instruction for manufacturing the insole relates to a data file and/or a 3D printing instruction prepared in a 3D printing-related format as known to the skilled person, e.g., STL, 3D Manufacturing Format (3MF), Additive Manufacturing File Format (AMF), PLY, Wavefront .obj, X3D.

In embodiments, pressure measurement is associated with automated sensor modules which perform measurements related to the user's foot sole. Examples of sensor modules are foot pressure recordings, foot pressure scans, digital pressure sensors, 2D or 3D foot scanners, pressure mat, video analysis. Still more examples are the devices described in US 10,463,257 B2.

In embodiments, the insole consists of one part, made of one material.

In embodiments, the insole consists of more than one part, for example, a first part and a second part.

In embodiments, the insole comprises a first part and a second part, and said first part at least partially, preferably completely, consists of a first material and said second part at least partially, preferably completely, consists of a second material different from said first material.

As known to the skilled person, an insole consisting of several parts and/or several materials can better meet various needs of a user, and, in pathologies, the needs of a patient. This, however, leads to an increased complexity making automation of insole design difficult.

An insole made of multiple parts is modular in design, and comprises at least two, preferably at least three parts, which preferably correspond to as many different materials ("multi-material"). These parts can be interconnected in any way. In embodiments in which the insole consists of multiple parts, at least the first and second parts, preferably all parts, are connected by fasteners such as an adhesive and/or by thermal welding, preferably all by the same fasteners.

In embodiments, the insole relates to an insole comprising more than one part, and at least one part is manufactured using the method according to the invention.

In embodiments, the insole relates to an insole comprising more than one part, and more than one part, preferably all parts, is manufactured using the method according to the invention. In such embodiments, the instruction for manufacturing the insole may include respective partial instructions, each of which is intended for separate devices for manufacturing the respective part of the insole, and wherein the parts are assembled only later. In preferred embodiments, the manufacturing of the respective parts is at least partially integrated, wherein one or more parts are produced in an integrated manner, for example by deposition of a second material for forming a second part on top of a first part comprising a first material.

In exemplary embodiments, the algorithmic conversion of pressure measurement and diagnosis to interpolating surface is associated with one or more of the following:
(1) difference in leg length
(2) anti-valgus wedge for partial or total foot length
(3) anti-varus wedges for partial or total foot length
(4) support of sustentaculum tali
(5) support of os cuboideum
(6) medial foot arch support (low bent foot / high bent foot)
(7) lateral foot arch support.

In preferred embodiments, the interpolating surface is transcoded into a production medium-dependent voxel representation, after which further modulation of the upper surface is applied. For example, the modulation may be associated with one or more of the following:
(a) midfoot support
(b) pressure relief zones
(c) material properties by zone.

In the following, the invention will be described using non-limiting examples illustrating the invention, which are not intended or to be interpreted to limit the scope of the invention.

### EXAMPLES

Although Examples 1-5 can be considered individually, they can also be understood coherently. For that reason, reference marks referring to figures belonging to another example are sometimes used in the various examples. Such reference marks are to be considered non-limiting to the respective example, and serve merely to illustrate the coherence of the examples.

### EXAMPLE 1: EXEMPLARY EMBODIMENT OF AN INSOLE DESIGNED ACCORDING TO THE INVENTION

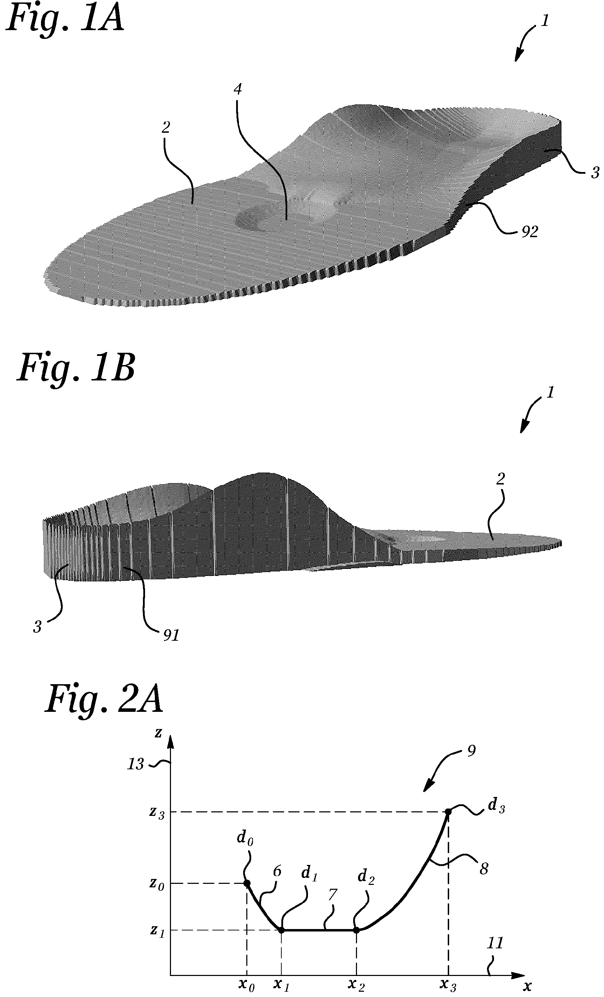

Fig. 1 shows an exemplary embodiment of an insole designed with a method according to the invention. Thereby, Fig. 1A shows a front view in perspective, and Fig. 1B shows a side view in perspective.

Insole 1 is designed for a shoe (not shown) and includes a smooth upper surface 2 and a side surface 3 extending according to a variable height between the lower surface (not shown) and the upper surface, all adapted to the anatomy of the user. In this example, the lower surface is flat, in accordance with information contained in the received sole profile indicating that a flat finishing of the lower surface is appropriate. The side surface, in turn, has been dimensioned taking into account information contained in the sole profile regarding the maximum outer contour within which the insole should fit, with a certain margin being observed to ensure proper fit. The variable height of the side surface, in this case roughly coinciding with the highest points of the insole, is dimensioned taking into account information contained in the sole profile regarding the maximum free height in the shoe.

The side surface 3 shows a lateral side 92 and a medial side 91, which extend between the extreme front tip and extreme heel tip of the insole, which, in this example, lie on the major axis of the insole (not shown). Moreover, in this example, the major axis of the insole coincides with the major axis 5 of the foot profile 50, which is not shown for Example 1 but is illustrated by Example 3. In variants of this example (not considered), these axes do not coincide. Thereby, the lateral side 92 thus extends between the same two points as the lateral side 52 of the foot profile 50, and the medial side 91 extends between the same two points as the medial side 51 of the foot profile 50 (compare: example 3), but not with the same shape, since the overall contour of the foot profile is narrower than that of the sole profile, with smaller length, smaller width and smaller inner surface.

The smooth upper surface shows a recess in a medial part, referred to as projecting member 4, which is adapted in shape, depth and position to the anatomy of the user.

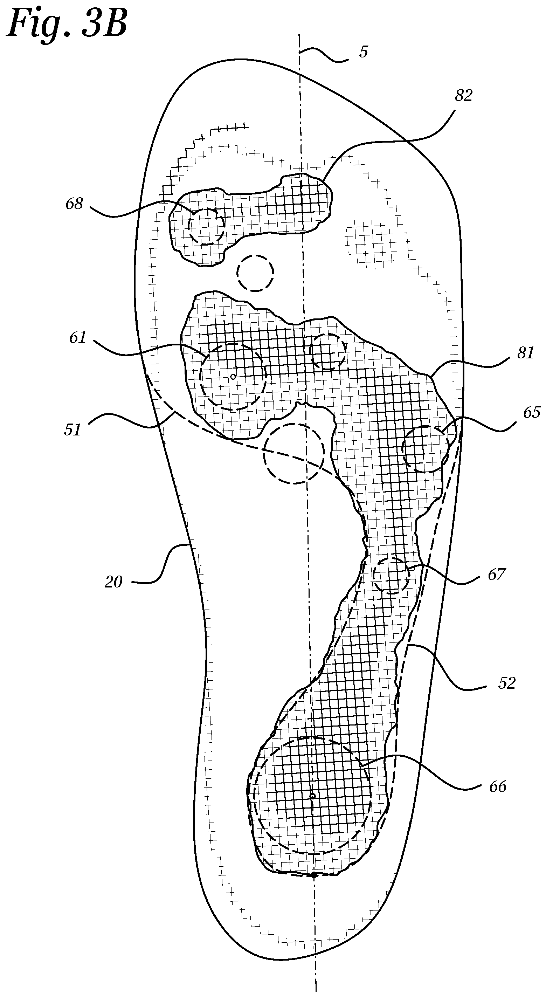

The insole was designed and manufactured using the method according to the invention, by means of 3D printing. This is based on a 2D pressure measurement based on inks, similar to that illustrated in Example 3, Fig. 3B. At the same time, the sole profile was also received.

The 2D pressure measurement makes it possible to map out the user's anatomy and adjust the insole accordingly. The sole profile, on the other hand, indicates the boundary conditions that the insole must meet to allow a good transition between the sole of the foot and the shoe, while ensuring that the insole properly fits in the shoe (sufficient free space all around, but not too loose).

The measurement is employed to determine, by means of computer vision, various anatomical zones, similar to zones 61, 65-68, 81-82 shown in Figure 3B of Example 3. In particular, the position of the first and fifth metatarsophalangeal, MTP, joints was taken into account, see for illustration reference marks 61, 65 in Figure 3B of Example 3.

Then, based on these anatomical zones, the major axis 5 is determined along with the foot profile, which in this example coincides with a contour 50 of anatomical zones, as illustrated in Example 3. In variants of this example, the foot profile includes other information in addition to this contour, such as one or more of the anatomical zones, or all of the anatomical zones detected.

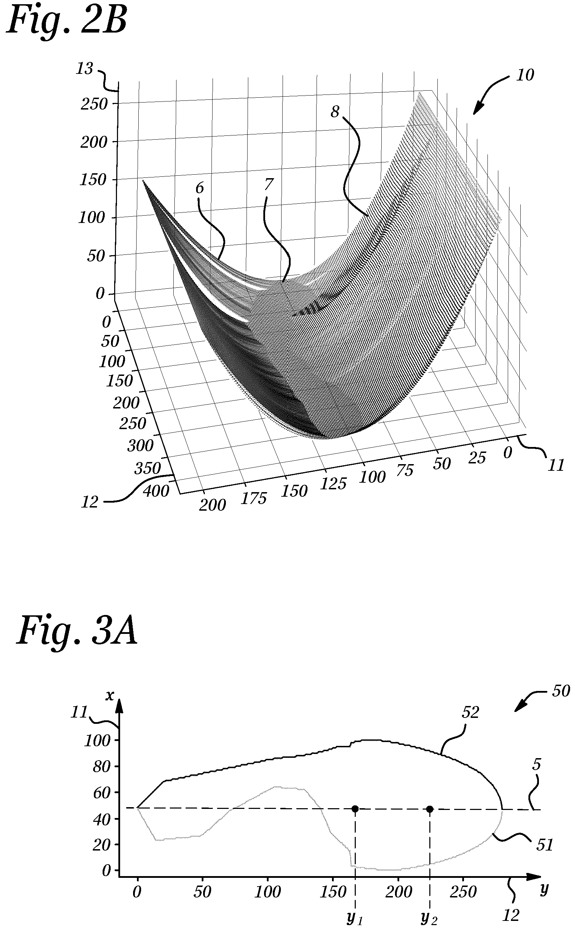

Subsequently, at least based on the foot profile 50 and the anatomical zones, an interpolating surface 10 is determined, similar to the one illustrated in Example 2, Fig. 2B. Here, anchor points d0-d3 are defined with anchor coordinates which have been calculated in accordance with the pressure measurement, thus ensuring a good fit of the surface to the user's anatomy. Then, the interpolating surface is created through these points, using a point cloud, applying a surface reconstruction algorithm associated with triangulation, and imposing certain boundary conditions on the surface, such as imposing a "derivative equal to zero" at anatomically selected locations, which translate into local maxima of the smooth interpolating surface.

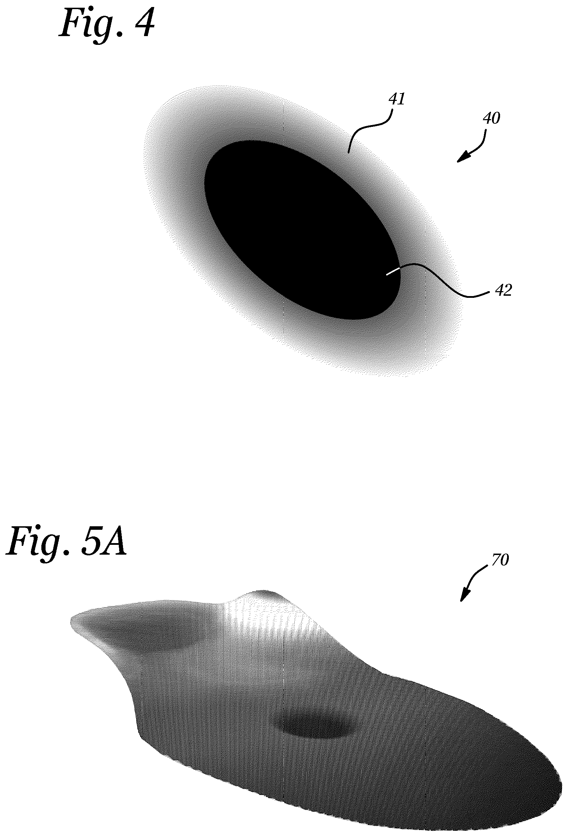

The interpolating surface thus obtained is then aligned with the sole profile to obtain an aligned 3D profile similar to the 3D profile 70 illustrated in Figs. 5A-5C of Example 5. In this example, the alignment of sole profile and interpolating surface is related to a coinciding major axis, but for other combinations of the sole profile and interpolating surface this does not have to be the case. When aligning, a portion in the shape and size of the sole profile is "cut out" from the interpolating surface.

The aligned 3D profile thus obtained is already smooth upwards at the edges of the upper surface, in accordance with the position and distribution of the anchor points when determining the interpolating surface. However, the aligned 3D profile is not yet provided with a projecting member 4.

In a next step, the aligned 3D profile is transcoded to a production medium-dependent voxel representation. In this example, this representation essentially concerns a conversion, from a mathematically defined surface, to a 2D grid of height values (not shown), which corresponds to a lossless bitmap representation. This representation is already well adapted to the production medium, being 3D printing. A further advantage is that this representation can easily be used for modulation of the upper surface. In this example, the modulation involves introducing a projecting member 4 as a 3D element. This projecting member 4 is also represented with a 2D grid of height values, which can be visually represented as grey scales. This is similar to the 3D element illustrated in Fig. 4 (Example 4), which is also a projecting member. Because this 3D element 40 is represented by a lossless bitmap representation, modulating is nothing more than the convolution of the lossless bitmap representation of the upper surface with the lossless bitmap representation of the 3D element. In this example, this involves correctly positioning the projecting member relative to the upper surface, followed by a position-by-position product of the values of both representations, to obtain the modulated upper surface.

In a next step, based on the aligned 3D profile 70 with finally modulated upper surface, an instruction is generated for manufacturing the insole 1. In this example, this is realized in real time, with the instruction being generated in software and sent to a 3D printer, which manufactures the entire insole accordingly.

### EXAMPLE 2: EXEMPLARY EMBODIMENT OF THE INTERPOLATING SURFACE

Fig. 2 shows an exemplary embodiment of an interpolating surface obtained when designing an insole according to the invention. Thereby, Fig. 2A shows an example of a section transverse to major axis 5. This 2D section extends along an x-direction 11 and a z-direction 13, while the major axis 5 extends along the y-direction 12. Fig. 2B shows the interpolating surface in perspective, with x-, y- and z-directions (11,12, 13).

The role of the interpolating surface 10 in the design of an insole 1 can be understood from Example 1. The interpolating surface 10 is a translation of the measurement of pressure of the sole of the foot. Thereby, the sole profile may (or may not) additionally already be taken into account in the determination of the interpolating surface since the sole profile defines the boundary conditions within which the insole must ultimately fit.

In this example, the initial interpolating surface 10, with 2D section 9 as illustrated in Fig. 2A and 3D representation as illustrated in Fig. 2B, is a surface with smooth lateral slope 6 between anchor points d0 and d1, passing into a flat part between anchor points d1 and d2, in turn passing into a smooth medial slope 8 between anchor points d2 and d3. This is an initial shape, where the interpolating surface exhibits a flat medial part over the entire major axis, because the abscissa of d1 and d2 are kept equal everywhere for the sake of simplicity (abscissa of d1 is z1, abscissa of d2 is also z1). In this example, however, the corresponding aligned 3D profile is still subjected to modulations, as explained for example in Example 1 (with reference to Example 4), so that the resulting insole shows a smooth medial part along part of the length of the major axis. In a variant of this example (not shown, but discussed in Example 1), the abscissae of d1 and d2 are not kept equal but are also used as additional degrees of freedom, with the values calculated as a function of measurement.

In this example, the interpolating surface 10 is determined according to a 3D characterization according to respective positions y1, y2 on the major axis 5, as illustrated in Fig. 3A of Example 3. For each such position y1, y2, anchor coordinates are determined. The anchor coordinates are determined using the anatomical zones determined from the measurement, as illustrated for example in Example 3 and discussed in Example 1. In this example, a foot profile 50 having a major axis 5 is determined from the anatomical zones, similar to those illustrated in Example 3, and the respective values of the lateral side 52 and the medial side 51 of the foot profile are used as respective ordinates x1 of d1 (lateral side) and x2 of d2 (medial side).

In this example, the ordinates x0 and x3 of anchor coordinates d0 and d3 are predetermined and are not related to the measurement. On the contrary, the interpolating surface extends between these predetermined fixed boundaries x0, x3. Therefore, this interpolating surface corresponds to an incompletely aligned 3D profile, and the application of the sole profile occurs only later, during alignment, when a portion the size of the sole profile is "cut" from the interpolating surface 10.

The abscissae z0 and z3 (of d0 and d3) are, however, not predetermined in this example, but calculated based on the anatomical zones and intensities of pressure in these zones. Thereby, the lateral and medial slopes are advantageously smooth, in accordance with the characteristics of the calculated interpolating surface, which allows a good connection with the sole of the foot and a good transfer of pressure, from the sole of the foot to the insole.

As will be clear from Fig. 2A, the 3D characterization in this example is realized with rectangular cross sections extending in the x-direction 11 between the same pair of predetermined values (ordinates x0, x3), and the actual 3D characterization is based on only four coordinate-related values: a pair of ordinates x1, x2, and a pair of abscissae z0, z3. As will be clear, this is an elegant and compact representation, allowing fast calculations and thus fast design.

In this example, the distance between different positions (granularity) is at least 1 mm, which means that less than 500 positions are sufficient for describing a full foot length. In this example, only four values are saved per position (the coordinate-related values), meaning that less than 2000 values need to be fit to the measurement of pressure. Such a lightweight representation is extremely compact and prevents overfitting when matching measurement and model. In addition, this representation is conveniently clear, and therefore easily manipulable by an operator.

### EXAMPLE 3: EXEMPLARY EMBODIMENT OF THE DETERMINATION OF A FOOT PROFILE AND ANATOMICAL FEATURES BASED ON MEASUREMENT

Fig. 3 illustrates an exemplary embodiment in which the foot profile 50 and various anatomical features, specifically anatomical zones (61, 65-68, 81-82), are determined based on a measurement. As explained in Example 1, this foot profile and these anatomical zones can be used to determine the interpolating surface, such as the interpolating surface illustrated in Example 2.

Fig. 3B thereby illustrates a 2D ink-based pressure measurement. This allows mapping of the user's anatomy.

The measurement is used to detect various anatomical zones 61, 65-68, 81-82 by means of computer vision. Detected zones 61 and 65 involve the position of the first and fifth metatarsophalangeal, MTP, joints, which play a crucial role in pressure distribution, and thus in the anatomically appropriate design of the insole. Detected zone 66 relates to the heel zone. Detected zone 68 relates to "apex one." Detected zone 67 relates to "base five" which can be used for further detection, by means of the computer vision algorithm, of the "lis franc".

In addition to these zones, the algorithm also detects cluster zones 81 and 82, wherein cluster zone 81 includes all five MTP joints, including the two extremities 61 and 65, as well as zones 67 and 66. Cluster zone 82 is located near the toes, and includes, among others, "apex one".

There is also the zone of foot contour 20, which is an estimate of the contour of the entire foot.

Based on these zones, the algorithm is able to determine a major axis 5, and also to obtain the medial side 51 and the lateral side 52 of the foot profile 50, both with the same starting and ending point on the major axis. This foot profile 50 is determined by the algorithm as an "enclosure" of zones; at the forefoot it concerns an enclosure of the foot contour, which at the level of the midfoot gradually changes into an enclosure of the cluster zone 81, up to the heel.

The resulting foot profile 50 is shown in Fig. 3A. With this foot profile 50, and the major axis 5 determined thereby, transverse cross sections can be defined based on respective positions y1, y2, for obtaining the interpolating surface, as explained in Example 1 and Example 2.

### EXAMPLE 4: EXEMPLARY EMBODIMENTS OF A 3D ELEMENT

Fig. 4 shows an example of a 3D element 40 for designing a projecting member. Element 40 comprises an annular section 41 with a gradual gradient of grey scales towards an oval core 42 exhibiting the same grey scale (black).

As explained in Example 1, this element 40 can be used for providing a projecting member 4, like the one in Example 1. The element 40 itself is represented with a 2D grid of height values, which are visually represented as grey scales. Because this 3D element 40 is represented by a lossless bitmap representation, it can be used for modulating the upper surface of the insole to be designed. It can be used for modulating the aligned 3D profile, with a convolution of the lossless bitmap representation of the upper surface, on the one hand, with the lossless bitmap representation of the 3D element, on the other hand. As explained in Example 1, this requires correct positioning of the projecting member relative to the upper surface, made possible by the correct identification of the anatomical zones. This positioning is followed by a position-by-position product of the values of both representations, for obtaining the modulated upper surface.

### EXAMPLE 5: EXEMPLARY EMBODIMENT OF AN ALIGNED 3D PROFILE ACCORDING TO THE INVENTION

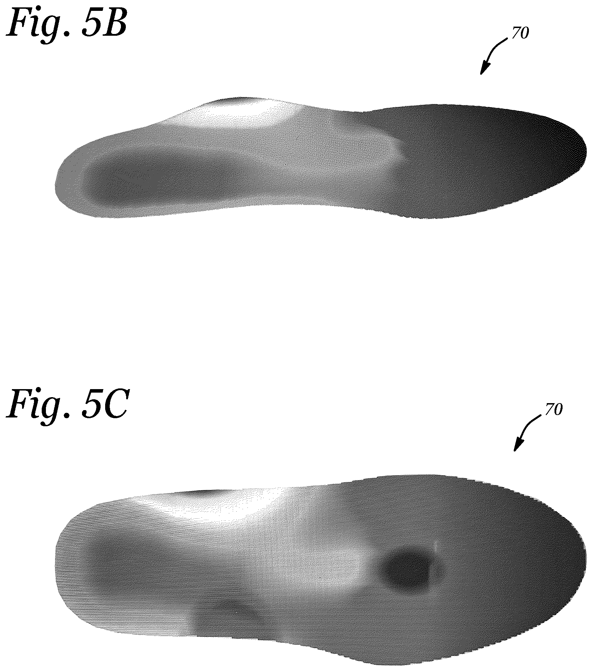

Fig. 5 shows an exemplary embodiment of an aligned 3D profile obtained when designing an insole according to the invention, according to various views in perspective. Thereby, Fig. 5A shows a lateral front view, Fig. 5B a side view, and Fig. 5C a top view.

As explained in Example 1, the aligned 3D profile 70 is obtained by aligning the interpolating surface with the sole profile. When aligning, a portion in the shape and size of the sole profile is "cut out" from the interpolating surface.

The aligned 3D profile thus obtained is already smooth upwards at the edges of the upper surface, in accordance with the position and distribution of the anchor points when determining the interpolating surface. Next, a projecting member 4 is added, by the modulation of the upper surface, for example as described in Examples 1 and 4. Then, an aligned 3D profile 70 with projecting member is obtained as shown in Figs. 5A-5C, which is ready for conversion into an insole generation instruction.

### (END OF EXAMPLE 5)

It is assumed that the present invention is not limited to the embodiments described above and that some modifications or changes can be added to the described examples without revaluing the added claims. For example, the present invention was described in Example 3 with pressure measurement using a 2D pressure measurement based on inks, but measurement using LIDAR is equally possible and is equally part of the invention.

## Claims

1. A method of designing an insole (1), comprising
receiving a measurement of a foot sole associated with pressure, said measurement preferably being obtained by placing a load on a measurement surface;
receiving a sole profile;
determining, based on said measurement, one or more anatomical zones (61, 65-68, 81-82);
determining, based on said anatomical zones (61, 65-68, 81-82), a foot profile (50) comprising a major axis (5);
determining, at least based on said foot profile (50) and said anatomical zones, an interpolating surface (10);
aligning the interpolating surface (10) with the sole profile to obtain an aligned 3D profile (70);
generating, based on said aligned 3D profile (70), an instruction for manufacturing the insole (1);
wherein said determination of the interpolating surface (10) is realized according to a 3D characterization according to respective positions (y1, y2) on said major axis (5);
wherein said determination of the interpolating surface (10) includes calculating, based on said foot profile (50) and said anatomical zones (61, 65-68, 81-82), a plurality of at least two anchor coordinates (d0-d3) each lying in a respective cross section corresponding to said respective positions (y1, y2), and calculating, based on said at least two anchor coordinates (d0-d3), an interpolating surface (10).

2. The method according to claim 1, wherein said determining of foot profile (50) comprises determining, based on said anatomical zones (61, 65-68, 81-82), said major axis (5), for further determination of said foot profile (50).

3. The method according to claim 1 or 2, wherein said 3D characterization relates to rectangular cross-sections extending in an x-direction between the same pair of ordinates (x0, x3); and wherein said 3D characterization is based on at least four coordinate-related values comprising a pair of ordinates (x1, x2) and a pair of abscissae (z0, z3) belonging to anchor coordinates (d0-d3).

4. The method according to claims 1 to 3, wherein said determination of the anatomical zones (61, 65-68, 81-82) based on the measurement relates to a computer vision algorithm, preferably a computer vision algorithm based on neural networks.

5. The method according to claim 4, wherein said determination of the anatomical zones includes determining, by means of the computer vision algorithm, at least one metatarsophalangeal, MTP, joint (61, 65).

6. The method according to claims 1 to 5, wherein said determination of the interpolating surface (10) is at least partly based on B-splines, preferably nurbs.

7. The method according to claims 3 to 6, wherein said incompletely aligned 3D profile relates to extending the interpolating surface (10) between predetermined fixed boundaries (x0, x3), and wherein said aligning relates to cutting out the sole profile from the interpolating surface (10).

8. The method according to claims 1 to 7, wherein said plurality of at least two anchor coordinates (d0-d3) per position each time comprises no more than ten anchor coordinates per position, preferably no more than five.

9. The method according to claims 1 to 8, wherein said plurality of at least two anchor coordinates (d0-d3) per position each relates to, across all positions, no more than 1,000 positions, preferably no more than 500 positions.

10. The method according to claims 1 to 9, wherein said determination of the interpolating surface (10) comprises the sub-steps:
calculating a point cloud;
applying, on the point cloud, a surface reconstruction algorithm, for obtaining the interpolating surface (10);
wherein said surface reconstruction algorithm relates to triangulation.

11. The method according to claims 1 to 10, wherein said method comprises **at the step** of determining the interpolating surface (10), **and/or at the step** of generating the instruction, the sub-step:
transcoding, from said interpolating surface (10) and/or said aligned 3D profile, to a production medium-dependent voxel representation;
wherein said generation of the instruction is realized based on the production medium-dependent voxel representation.

12. The method according to claims 1 to 11, wherein said method comprises **at the step** of determining the interpolating surface (10), **and/or at the step** of generating the instruction, the sub-steps:
receiving a 3D element (40) **characterized by** one or more element parameters;
modulating, based on said 3D element (40), said interpolating surface (10) and/or said aligned 3D profile, to obtain a modulated representation;
wherein said generation of the instruction is realized based on the modulated representation.

13. The method according to claim 12, wherein it is the step of generating the instruction that comprises the sub-steps of receiving and modulating; wherein the 3D element (40) is represented by a lossless bitmap representation; and wherein the modulating relates to the convolution of the lossless bitmap representation with a production medium-dependent voxel representation of the interpolating surface or the aligned 3D profile.

14. A device comprising a processor configured to perform a method according to claims 1 to 13.

15. A system comprising:
- the device according to claim 14 for generating an instruction for manufacturing the insole (1); and
- means for receiving said instruction and manufacturing the insole (1), via milling and/or 3D printing and based on said instruction.

16. Computer program product comprising memory comprising instructions which, when executed on a processor, cause the processor to implement a method according to claims 1 to 13.
